# EUROPEAN PATENT SPECIFICATION

(11) **EP 4 171 729 B1**
(45) Date of publication and mention of the grant of the patent: **06.05.2026**
(21) Application number: 21736697.0
(22) Date of filing: 11.06.2021
(51) Int. Cl.: A61N 1/378, A61N 1/36, A61N 1/05

(54) **SENSORY UNIT FOR DENTAL IMPLANTS**
SENSOREINHEIT FÜR ZAHNIMPLANTATE
UNITÉ SENSORIELLE POUR LES IMPLANTS DENTAIRES

(30) Priority: 24.06.2020 PT 2020116518
(43) Date of publication of application: 03.05.2023
(73) Proprietor: Universidade do Porto - Reitoria, 4099-002 Porto (PT); Mechanobionics Lda, 4050-012 Porto (PT)
(72) Inventor: PEIXOTO MACHADO DA SILVA, José Alberto, 4200-465 Porto (PT); MAGALHÃES MENDES, Joaquim Gabriel, 4200-465 Porto (PT); SOBRADO MARINHO, Jorge Serafim, 4050-012 Porto (PT)
(74) Representative: Couto, Cláudia
(86) International application number: PCT/IB2021/055143
(87) International publication number: WO 2021/260473

(56) References cited:
- EP-A1- 2 945 691
- EP-B1- 2 945 691
- US-A1- 2009 216 292
- US-A1- 2012 064 486
- US-A1- 2015 174 406
- BANI-HANI MUATH ET AL: "Energy harvesting from mastication forces via a smart tooth", PROCEEDINGS OF SPIE; [PROCEEDINGS OF SPIE ISSN 0277-786X VOLUME 10524], SPIE, US, vol. 9803, 20 April 2016 (2016-04-20), pages 98030A - 98030A, XP060068887, ISBN: 978-1-5106-1533-5, DOI: 10.1117/12.2219390

## Description

### Technical Domain

The present application describes a sensory unit for implant-supported dental implants or prostheses that will provide neuro-sensory information to the trigeminal nerve endings.

### Background

Previous studies related to the matter herein disclosed discussed the use of a bionic device for transducing masticatory pressure into an electrical stimulus, capable of being perceived by the body in the form of nociceptive stimulus, triggering on the part of the organism an adequate motor defense response or decrease of muscle contraction.

However, this type of devices did not contemplate the possibility of identifying the gradient of mechanical force exerted on the masticatory surfaces of the teeth of the implant-supported or implant-retained prosthesis, namely the modulation of the electrical signal produced from the mechanical forces exerted by the fixed prosthesis, on the abutment resting on the implant.

Therefore, and as a differentiating element in relation to the prior art, the present unit has the ability to better match light mechanical loads to a specific individualized signal. This fact is important, since it returns one of the important functions of natural teeth, which is to signal the position of the jaw as a determining factor to provide information to the structures of the cerebral cortex that allow a motor response to the beginning of gait movement, and to define the sequence of contraction of the various muscle groups in view of the coordinated movement of body displacement, inserted in a trajectory.

Prior art is known from BANI-HANI MUATH ET AL: "Energy harvesting from mastication forces via a smart tooth", PROCEEDINGS OF SPIE ISSN 0277-786X VOLUME 10524 as well as from US 2009/216292 A1, US 2015/174406 A1, EP 2 945 691 A1, EP 2 945 691 B1 and US 2012/064486 A1.

### Summary

The present application describes a sensory unit for dental implants according to claim 1. The sensory unit comprises at least one dental device responsible for transforming the mechanical forces from occlusion and mastication actions into electrical signals; and at least one stimulator device responsible for transforming electrical signals from the dental device into neuronal stimuli; wherein the neuronal stimuli promote the restoration of the proprioceptive, nociceptive and stereognostic sensitivity of the user.

In a proposed embodiment, the at least one dental device transmits the electrical signals to the at least one stimulator device.

In yet another possible embodiment, the electrical signals are transmitted from the at least one dental device to the at least one stimulator device via a leading wire system or via a wireless communication system.

In yet another possible embodiment, the at least one stimulator device applies neuronal stimuli to the nerve endings of the maxillary or mandibular trigeminal bundles. In yet another possible embodiment, the at least one dental device comprises the use of a sealed titanium encapsulation for the incorporation of all constituent elements.

In yet another possible embodiment, the constituent elements of the at least one dental device comprise the use of biocompatible force sensing material, a battery, an signal acquisition and processing circuit, an integration module, and a transmitter module.

In yet another possible embodiment, the at least one stimulator device comprises the use of an airtight biocompatible coating that incorporates all constituent elements.

In yet another possible embodiment, the constituent elements of the at least one stimulator device comprise the use of a receiver, a battery, an integration module, a processing module, a conductive filament, a conductive housing with biocompatible coating and a conductive filament incorporated in the conductive housing and interface with "cuff" type electrodes.

In yet another possible embodiment, the at least one dental device can be installed in several locations comprising the dental implant, the prosthetic abutment, the prosthetic device that integrates the prosthetic abutment, occlusal surface of the fixed prosthesis teeth or yet in the space reserved for the screw well that fastens the abutment to the implant head.

In yet another possible embodiment, the at least one stimulator device can be installed in several locations comprising the vicinity of the terminal branch, respectively of the infraorbital nerve, in the upper maxillary nerve branch, or in the vicinity of the mentonian nerve, in the lower maxillary or mandibular nerve branch.

### Brief description

The present application describes a sensory unit for dental implants or prostheses and aims at returning tooth sensitivity lost as a result of tooth extraction, devitalization, ablative oral surgery, and infections that are the cause for neurophysiological disturbances in stomatognathic system functioning.

Therefore, the present invention aims at restoring the neurosensitive connection between the teeth, or their substitutes, whether they are implants, cemented crowns or screwed to implants or any other mechanical device related thereto, and the brain, making it possible to be applied on abutments, dental implants, crowns, or prosthetic structures mechanically related thereto. The problem that this solution allows to overcome is the lack of sensitivity that patients rehabilitated with fixed prostheses on implants complain about. This lack of sensitivity causes a deficit in the ideation of the masticatory model, as well as changes in the perception of the dimension, hardness, and texture of food, thus contributing to a significant loss of the ability to taste food. The loss of dental sensitivity, when causing a decrease in the sensitive input of proprioception and stereognosis, indirectly decreases the abilities of body posture, balance and locomotion while walking and running.

The present unit then makes it possible to contribute to the reestablishment of proprioceptive, nociceptive and stereognostic sensitivity, either diminished or lost due to teeth extraction and/or devitalization.

In this way, the superior functions are improved as a consequence of the restoration of related sensitivities, namely:
- Control of the entire mastication process with modulation of the force exerted during the masticatory process;
- Food tasting during the masticatory process;
- Contribution to the detection and treatment of occlusion disorders called parafunctional masticatory disorders;
- Contribution to a better information of the brain on the relative position of the body in the surrounding area, and thereby facilitate the brain's automatic mechanisms of posture, balance and gait.

Individuals who have this unit implemented in their mouth will be able to perform more differentiated masticatory cycles, in lesser numbers and with greater efficiency, as the information that reaches the brain is more detailed about the hardness, texture, dimension, and taste of the foods that have been placed in the mouth.

This unit thus makes it possible to better discern the masticatory model for each class of food. On the other hand, it will also improve the perception of food hardness, preventing masticatory overloads, and therewith, minimizing the occurrence of fractures of fixed prostheses, so frequent in this type of prosthetic devices.

Acting in complementarity, these units may also provide and act as a tool for detecting and preventing parafunctional masticatory movements, commonly known as bruxism.

The benefits of the present technique are evident and can be applied in the clinical field of stomatology, dental medicine, maxillofacial surgery, physical medicine and rehabilitation, gerontology, sports medicine, among others.

### Brief Description of The Drawings

For an easier understanding of the present application, figures are herein attached, which represent embodiments which however are not intended to limit the art herein disclosed.

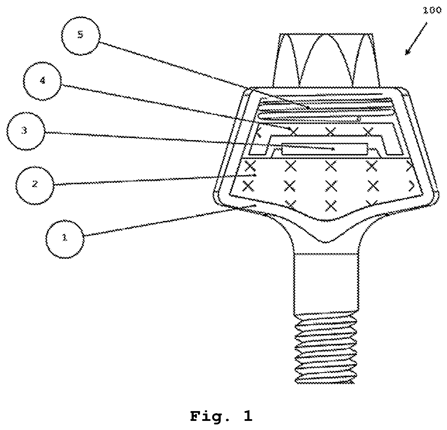

Figure 1 illustrates the first device (100), or dental device, comprising the following elements:
   1 - Device housing in biocompatible force sensing material;
   2 - Battery;
   3 - signal acquisition and processing circuit;
   4 - Integration module;
   5 - Transmitter / Antenna.

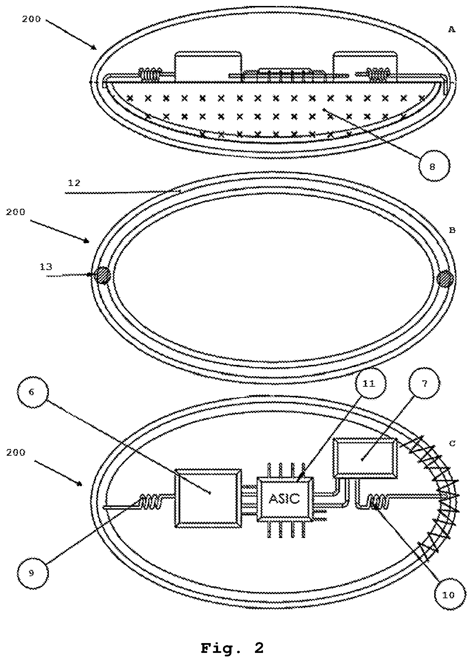

Figure 2 illustrates the second device (200), or stimulator device, wherein A represents the vertical section view of the device, B represents the top view of the device and C represents the horizontal section view of the device, comprising the following elements:
   6 - Integration module;
   7 - Module for processing and producing the neural stimulus;
   8 - Battery;
   9 - Receiver / Antenna;
   10 - Conductive filament of neural stimulus for surrounding tissues;
   11 - signal acquisition and processing circuit;
   12 - Conductive housing with biocompatible coating;
   13 - Conductive filament incorporated in the conductive housing and interface with "cuff" type electrodes.

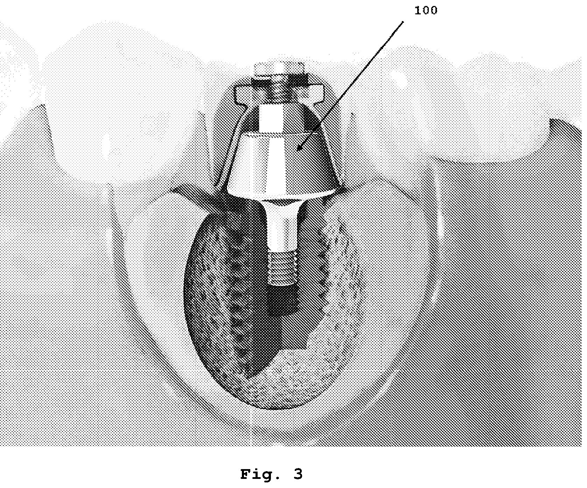

Figure 3 illustrates the incorporation of the dental device (100) in a dental arch.

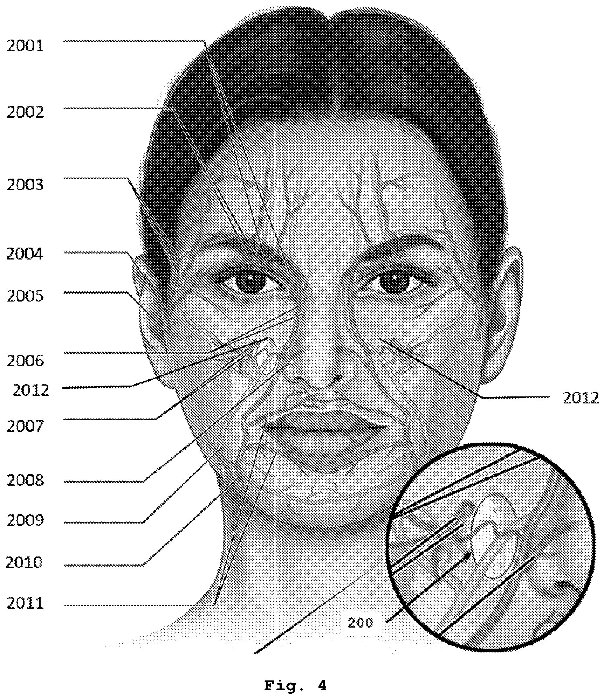

Figure 4 illustrates the incorporation of the stimulator device (200) for sensing the oral cavity, in the vicinity of the end branch, wherein:
   2001 - supratrochlear artery and vein;
   2002 - supraorbital artery and vein;
   2003 - superficial temporal artery and vein;
   2004 - retromandibular vein;
   2005 - external carotid artery;
   2006 - angular artery and vein;
   2007 - infraorbital artery and vein;
   2008 - lateral nasal artery;
   2009 - facial vein;
   2010 - facial artery;
   2011 - labial arteries;
   2012 - infraorbital nerve foramen.

### Description of the embodiments

Referring to the figures, some embodiments are now described in more detail, which are not intended, however, to limit the scope of the present application.

The present application describes a sensory unit composed of two or more subunits, or devices. The set of the two devices that act in sequence, provides restitution of masticatory dental sensitivity, and proprioceptive dental sensitivity.

The first functionality replaced by the developed unit is important for the functions of grasping, mastication, swallowing food, and controlling occlusal overloads. The second is to restore the dental sensitivity established by small contacts between the dental cusps, and which allow the brain to receive spatial information about the position of the jaw and thereby improve the functions of body posture, gait and running.

One of the devices, hereinafter referred to as the dental device (100), as it is installed in the dental arch, can be incorporated in several locations comprising the dental implant, the prosthetic abutment, and also the prosthetic device that integrates the prosthetic abutment. The main purpose of the dental device (100) is to receive the mechanical loads from occlusion and mastication, and to transform them into electrical signals. These electrical signals will in turn be transmitted to another device, called a stimulator device (200), said transmission can be carried out through a leading wire system, or through a wireless communication system, through the most appropriate biocompatible method for the relevant case.

The dental device (100), therefore, has the function of transforming the mechanical loads into electrical signals that will then be transmitted to the stimulator device (200).

In one of the proposed embodiments, the dental device (100) is installed in the dental arch, being installed between the abutment and the implant, and has a component that allows the interface between the implant and the abutment, the housing (1) being made up of a biocompatible force sensing material. The remaining constituent components of the dental device (100) will also comprise a battery (2), an signal acquisition and processing circuit (3), an integration module (4) and a transmitter (5) that could be a micro antenna. All these components will be grouped and included in an abutment with a conical, straight or angled profile. The material responsible for sealing and encapsulating all these components, incorporating them in a single compartment, is titanium in one of the proposed embodiments. The signal acquisition and processing circuit (3) is responsible for processing and sending the signals from the force sensing housing (1) to the integration module (4). The integration module (4) is responsible for receiving the electrical signals and sending thereof to the antenna (5) in order to be transmitted to the neuronal stimulation unit (200) .

The dental device (100), in one of the proposed embodiments, is mostly made of titanium, and has an extended dimension in the form of a "multi-unit" abutment, incorporating inside all electronic components necessary for the detection of mechanical forces of dental occlusion and transformation thereof into a modulated electrical signal. The detected forces will have an amplitude between 0.2N and about 180N.

The dental device (100), therefore, has the particularity of being able to be installed in the region of the occlusal surface of the teeth of the fixed prosthesis, or else, in a more miniaturized form and at micro scale, in the space reserved for the screw well that tightens the abutment to the implant head.

In terms of dimensions, and in one of the proposed implementation forms, the dental device (100) comprises diameters between 1mm and 5mm, preferably between 1.5mm and 4.3mm, and a total height of approximately 6.5mm.

The stimulator device (200) consists of several components grouped within an airtight biocompatible coating (12). Among these, an signal acquisition and processing circuit (11), a receiver (9), which may be a micro antenna and a signal amplifier circuit, a battery (8), an integration module (6), a processing module (7) and generation of neural stimuli, and a conductive filament of neural stimulus for surrounding tissues (10) which is incorporated in the conductive housing (13) can be part of one of the proposed embodiments. The stimulator device (200) will be installed in the vicinity of the end branch, respectively of the infraorbital nerve, in the upper maxillary nerve branch, or in the vicinity of the mentonian nerve, in the lower maxillary or mandibular nerve branch. The conductive filament of neural stimulus for surrounding tissues (10) is composed, in one of the proposed embodiments, by a chromium-cobalt-nickel alloy (MP35N) or by a platinum-iridium alloy.

The stimulator device (200) is constructed of biocompatible and flexible material, and can be coated in materials derived from polyfluorethanes, including all electronic components (at micro or nano scale) necessary for the production of neuronal stimulus, or neural stimuli in continuous mode, pulse, or sequence of pulses, with an electric current intensity that can vary between a few hundred microamperes and a few milliamperes with variable frequency between 5 to 100hz, and a duration of a few hundred microseconds.

In one of the proposed embodiments, the receiver (9) of the stimulator device (200) will be responsible for receiving the signal from the dental device (100), ensuring the delivery thereof to the integrating component (6) for the purpose of being transformed by the signal acquisition and processing circuit (11) in a type of neurological signal that crosses the coating (12) of the stimulator device (200), and excites the nerve endings in the vicinity. Among the remaining components of the stimulator device (200), it is also possible to list the conductive stimulation filament (13) which will be embedded in the coating of the stimulator device (200). The integration module (6) is, therefore, responsible for the amplification of the electrical signal from the dental device (100), demodulating the signal, and its preliminary processing. The amplification and neural stimulus production module (7) is responsible for receiving the signal from the integration module (6), and modulating and amplification the neuronal stimulus. Finally, the conductive housing with a biocompatible coating (12) ensures hermetic encapsulation of the entire microsystem.

The stimulator device (200) will therefore be responsible for receiving electrical signals from the dental device (100), thus modeling, processing and transforming them into neural stimuli that will be transmitted through the tissues in the vicinity of nerve endings, to the nerve bundle endings. This signal is generated in the receiving device itself, and will be transmitted for application in the nerve ending of the branch at bundle V2 or V3 of the maxillary sensitive trigeminal bundle and mandibular sensitive trigeminal bundle. This nerve stimulation, will make its way to the brainstem, and therefrom to the posterior thalamic nuclei, going up to the sensitive cerebral cortex, being then due to the phenomenon of cerebral neuroplasticity, interpreted as a neurological signal coming from the masticatory region, being processed as such by the brain to provide a motor, masticatory or postural response, or an integrative response through the cerebral amygdala or the hippocampus, with the generation of mood changes, or stimulation of cognitive functions, such as memory and concentration.

Still in relation to the characteristics of the stimulator device (200), and in one of the proposed embodiments, it has approximate dimensions of 6.0mm in width and 2.5mm in height.

The present description is of course in no way restricted to the embodiments presented herein and a person of ordinary skill in the art may provide many possibilities of modifying it without departing from the general idea as defined in the claims. The preferred embodiments described above are obviously combinable with each other. The following claims further define preferred embodiments.

## Claims

1. Sensory unit suitable for dental implants or prostheses to promote the reestablishment of at least one of proprioceptive, nociceptive and stereognostic sensitivity in a user, comprising
at least one dental device (100), configured to be installed in a location of a dental arch of the user, adapted to transform mechanical forces from occlusion and mastication actions from a user into electrical signals; and
at least one stimulator device (200); **characterized in that**
the at least one stimulator device (200) comprises a biocompatible airtight coating (12) incorporating a receiver (9), a battery (8), an integration module (6) and a processing module (7) for producing a neural stimulus, and comprises a conductive filament (10) incorporated in a conductive housing with the biocompatible coating (12) and interfacing with cuff type electrodes (13), the at least one stimulator device (200) being configured to transform the electrical signals received from the dental device (100) into neuronal stimuli that are transmitted through the biocompatible coating (12) to enable the controlled excitation of said nerve endings, wherein the neuronal stimuli comprise continuous mode, pulse or sequence of pulses with an electric current intensity varying between a few hundred microamperes and a few milliamperes, with a variable frequency between 5 to 100 Hz, and a pulse duration of a few hundred microseconds.

2. Sensory unit according to the preceding claim, **characterized in that** the at least one dental device (100) is configured to transmit the electrical signals to the at least one stimulator device (200).

3. Sensory unit according to any of the preceding claims, **characterized in that** the electrical signals are configured to be transmitted by the at least one dental device (100) to the at least one stimulator device (200) via a leading wire system or via a wireless communication system.

4. Sensory unit according to any of the preceding claims, **characterized in that** the at least one dental device (100) comprises a sealing and encapsulating titanium compartment for the incorporation of all constituent elements.

5. Sensory unit according to the preceding claim, **characterized in that** the constituent elements of the at least one dental device (100) comprises a biocompatible force sensing material (1), a battery (2), a signal acquisition and processing circuit (3), an integration module (4), and a transmitter module (5).

6. Sensory unit according to preceding claims 1 to 5, **characterized in that** the at least one dental device (100) is configured to be installed in different locations comprising the dental implant, the prosthetic abutment, the prosthetic device that integrates the prosthetic abutment, occlusal surface of the fixed prosthesis teeth or in the space reserved for the screw well that tightens the abutment at the implant head.

7. Sensory unit according to preceding claims 1, 2, 3, and 6, **characterized in that** the at least one stimulator device (200) is configured to be installed in several locations comprising the vicinity of the terminal branch, respectively of the infraorbital nerve (2012), in the upper maxillary nerve branch, or in the vicinity of the mentonian nerve, in the lower maxillary or mandibular nerve branch.

## Patentansprüche

1. Sensoreinheit geeignet für Zahnimplantate oder Prothesen zur Unterstützung der Wiederherstellung mindestens einer der folgenden Sensibilitäten beim Anwender: propriozeptive, nozizeptive und stereognostische Sensibilität, umfassend mindestens eine zahnmedizinische Vorrichtung (100), die dazu konfiguriert ist,
an einer Position eines Zahnbogens des Anwenders installiert zu werden, wobei die Vorrichtung dazu eingerichtet ist, mechanische Kräfte aus Okklusions- und Mastikationsvorgängen des Anwenders in elektrische Signale umzuwandeln; und
mindestens eine Stimulationsvorrichtung (200);
**dadurch gekennzeichnet, dass**
die mindestens eine Stimulationsvorrichtung (200) eine biokompatible, luftdichte Beschichtung (12) umfasst, der in einem Empfänger (9), einer Batterie (8), einem Integrationsmodules (6) und einer Verarbeitungsmoduls (7) für die Herstellung eines neuralen Reizes integriert ist, und einen leitfähigen Draht (10) umfasst, der in einem leitfähigen Gehäuse mit der biokompatiblen Beschichtung (12) integriert ist und mit Manschetten-Elektroden (13) in Verbindung steht, wobei die mindestens eine Stimulationsvorrichtung (200) so konfiguriert ist, dass die von der zahnmedizinische Vorrichtung (100) empfangenen elektrischen Signale in neuronale Reize umgewandelt werden, die durch die biokompatible Beschichtung (12) übertragen werden, um eine kontrollierte Erregung der genannten Nervenendigungen zu ermöglichen, wobei die neuronalen Reize einen kontinuierlichen Modus, Impuls oder Impulsfolge mit einer elektrischen Stromstärke von einigen hundert Mikroampere bis zu einigen Milliampere, einer variablen Frequenz von 5 bis 100 Hz und einer Impulsdauer von einigen hundert Mikrosekunden umfassen.

2. Sensoreinheit nach dem vorstehenden Anspruch, **dadurch gekennzeichnet, dass** die mindestens eine zahnmedizinische Vorrichtung (100) dazu konfiguriert ist,
die elektrischen Signale zu mindestens einer Stimulationsvorrichtung (200) zu übertragen.

3. Sensoreinheit nach einem der vorstehenden Ansprüche, **dadurch gekennzeichnet, dass** die elektrischen Signale dazu konfiguriert sind,
von der mindestens einen zahnmedizinischen Vorrichtung (100) an die mindestens eine Stimulationsvorrichtung (200) über ein Leitungssystem oder über ein drahtloses Kommunikationssystem übertragen zu werden.

4. Sensoreinheit nach einem der vorstehenden Ansprüche, **dadurch gekennzeichnet, dass** die mindestens eine zahnmedizinische Vorrichtung (100) eine Abdichtung und Verkapselung des Titanfachs zur Aufnahme aller Bestandteile umfasst.

5. Sensoreinheit nach einem der vorstehenden Ansprüche, **dadurch gekennzeichnet, dass** die Bestandteile von mindestens einer zahnmedizinischen Vorrichtung (100) ein biokompatibles Kraftmessmaterial (1), eine Batterie (2), eine Signalaufnahme -und Verarbeitungsschaltung (3), ein Integrationsmodul (4) und ein Übertragungsmodul (5) umfassen.

6. Sensoreinheit nach den vorstehenden Ansprüchen 1 bis 5, **dadurch gekennzeichnet, dass** die mindestens eine zahnmedizinische Vorrichtung (100) dazu konfiguriert ist,
in verschiedenen Positionen installiert zu werden, wobei sie das Zahnimplantat, das prothetische Abutment, die prothetische Vorrichtung, die das prothetische Abutment integriert, die okklusale Oberfläche der festsitzenden Prothesenzähne oder den für den Schraubenkanal vorgesehenen Raum, der das Abutment am Implantatkopf befestigt, umfasst.

7. Sensoreinheit nach den vorstehenden Ansprüchen 1, 2, 3 und 6, **dadurch gekennzeichnet, dass** die mindestens eine Stimulationsvorrichtung (200) dazu konfiguriert ist,
in mehreren Positionen installiert zu werden, umfassend der Nähe des Endastes bzw. des Infraorbitalnervs (2012), im Ast des oberen Oberkiefernervs oder in der Nähe des Kinnnervs, im Ast des unteren Oberkiefer- oder Unterkiefernervs.

## Revendications

1. Unité sensorielle approprié pour les implants dentaires ou prothèses pour promouvoir le rétablissement d'au moins l'une des sensibilités proprioceptive, nociceptive ou stéréognosique chez un utilisateur, comprenant
au moins un dispositive dentaire (100), configuré pour être installé dans un
emplacement d'une arcade dentaire de l'utilisateur, adapté pour transformé les forces mécaniques provenant des actions d'occlusion et de mastication de l'utilisateur en signaux électriques ; et
au moins un dispositif stimulateur (200) ;
**caractérisé en ce que**
au moins un dispositive stimulateur (200) comprend un revêtement étanche biocompatible (12) incorporant un récepteur (9), une batterie (8), un module d'intégration (6) et un module de traitement (7) pour produire un stimulus neuronal, et comprend un filament conducteur (10) incorporé dans un boîtier conducteur avec le revêtement biocompatible (12) et interagissant avec des électrodes de type manchon (13), au moins un dispositif stimulateur (200) étant configuré pour transformer les signaux électriques reçus du dispositif dentaire (100) en des stimuli neuronaux qui sont transmis à travers le revêtement biocompatible (12) afin de permettre l'excitation contrôlée desdites terminaisons nerveuses, dans laquelle les stimuli neuronaux comprennent un mode continu, une impulsion ou une séquence d'impulsions avec une intensité de courant électrique variant entre quelques centaines de microampères et quelques milliampères, avec une fréquence variable entre 5 et 100 Hz, et une durée d'impulsion de quelques centaines de microsecondes.

2. Unité sensorielle selon la revendication précédente, **caractérisée en ce que** au moins un dispositif dentaire (100) est configuré pour transmettre
des signaux électriques à au moins un dispositif stimulateur (200).

3. Unité sensorielle selon l'une quelconque des revendications précédentes, **caractérisée en ce que** les signaux électriques sont configurés pour transmettre
par au moins un dispositif dentaire (100) vers au moins un dispositif de stimulation (200) par un système de fils conducteurs ou par un système de communication sans fil.

4. Unité sensorielle selon l'une quelconque des revendications précédentes, **caractérisée en ce que** au moins un dispositif dentaire (100) comprend un compartiment en titane d'étanchéité et d'encapsulation pour l'incorporation de l'ensemble des éléments constitutifs.

5. Unité sensorielle selon la revendication précédente, **caractérisée en ce que** les éléments constitutifs du ou d'au moins un dispositif dentaire (100) comprend un matériau biocompatible de détection de force (1), une batterie (2), un circuit d'acquisition et de traitement de signal (3), un module d'intégration (4) et un module émetteur (5).

6. Unité sensorielle selon les revendications 1 à 5 précédentes, **caractérisée en ce que** au moins un dispositif dentaire (100) est configuré pour être
installé à différents emplacements comprenant l'implant dentaire, le pilier prothétique, le dispositif prothétique intégrant le pilier prothétique, la surface occlusale des dents de la prothèse fixe ou dans l'espace réservé au puits de vis qui serre le pilier sur la tête de l'implant.

7. Unité sensorielle selon les revendications 1, 2, 3 et 6 précédentes, **caractérisée en ce que** au moins un dispositif stimulateur (200) est configuré pour être
installé à plusieurs emplacements comprenant la proximité de la branche terminale, respectivement du nerf infraorbitaire (2012), dans la branche du nerf maxillaire supérieur, ou dans la proximité du nerf mentonnier, dans la branche du nerf maxillaire inférieur ou mandibulaire.
